(19)

**Europäisches Patentamt**

**European Patent Office**

**Office européen des brevets**

(11)  **EP 4 787 381 A1**

(12)  # EUROPEAN PATENT APPLICATION

(43) Date of publication:
**05.08.2026  Bulletin 2026/32**

(21) Application number: **25154581.0**

(22) Date of filing: **29.01.2025**

(51) International Patent Classification (IPC):
**G16C 20/10** (2019.01)

(52) Cooperative Patent Classification (CPC):
**G16C 20/10**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Bayer Aktiengesellschaft
51373 Leverkusen (DE)**

(72) Inventors:
 • **SCHNECKENER, Sebastian
   51373 Leverkusen (DE)**
 • **SCHURKEMEYER, Jan
   51373 Leverkusen (DE)**

 • **FÜHRER, Florian
   51373 Leverkusen (DE)**
 • **SCHMIDT, Sebastian
   51373 Leverkusen (DE)**
 • **OTT, Mark-Christoph
   51373 Leverkusen (DE)**
 • **PETERS, Onno
   51373 Leverkusen (DE)**

(74) Representative: **BIP Patents
c/o Bayer Intellectual Property GmbH
Alfred-Nobel-Straße 50
40789 Monheim am Rhein (DE)**

Remarks:
Claims filed after the date of filing of the application
(Rule 68(4) EPC).

(54)  **PREDICTION OF REACTION SEQUENCES**

(57)  Systems, methods, and computer programs disclosed herein relate to the prediction of reaction sequences.

**Fig. 6**

EP 4 787 381 A1

**Description**

FIELD OF THE DISCLOSURE

Systems, methods, and computer programs disclosed herein relate to the prediction of reaction sequences.

BACKGROUND

A reaction sequence refers to a series of consecutive chemical reactions where the product of one reaction becomes the reactant in the next.

Such reaction sequences play an important role in many areas.

One example is the breakdown of a chemical substance during wastewater treatment.

In the field of wastewater treatment, a reaction sequence refers to a series of chemical, physical, and/or biological processes designed to remove contaminants from water, making it suitable for discharge back into the environment or for reuse. Wastewater treatment is a multi-stage process, and the specific sequence of reactions and treatments depends on the composition of the wastewater and the quality requirements of the treated water. It could be advantageous to be able to predict the reaction sequence of such a contaminant when passing through one or more treatment stages of a wastewater treatment plant.

Another example of a reaction sequence is the fate of a substance introduced into the environment. For example, understanding the reaction sequences of crop protection products, industrial chemicals, and other substances released into the environment is important for assessing their ecological impact. Some substances may not be inherently toxic but may be transformed into harmful products, others may be persistent or may cause adverse effects due to accumulation. Identifying reaction products of reaction sequences is critical for assessing the overall impact of a substance on target organisms, off-target organisms and/or ecosystems.

Another example of a reaction sequence is the metabolism of a xenobiotic. Metabolism is a set of life-sustaining chemical reactions in an organism. Main functions of metabolism are (i) the conversion of energy in food to energy available to run cellular processes; (ii) the conversion of food to building blocks of proteins, lipids, nucleic acids, carbohydrates and/other substances that fulfil a function for the organism; and (iii) the elimination of metabolic wastes and/or detoxification processes.

A xenobiotic is a chemical substance that is foreign to a biological system. It comes from outside the biological system and is not naturally produced or expected to be present within the biological system. Most of drugs and crop protection products are xenobiotics: synthetic compounds which do not or rarely exist as natural products and/or contain structural elements that cannot be synthesized biochemically (see, e.g., P.G. Rieger et al.: Xenobiotics in the environment: present and future strategies to obviate the problem of biological persistence, Journal of Biotechnology, Volume 94, Issue 1, 2002, Pages 101-123).

Understanding which metabolites can arise when a xenobiotic is metabolized in a target and/or off-target organism as well as the ecosystem can be important for various aspects.

For example, in pharmaceutical development, identifying metabolites is crucial for drug safety and efficacy. It helps in predicting potential side effects, interactions with other substances, and the overall risk profile of a drug in the target and non-target organs and organisms. In pharmaceutical research, understanding the metabolism of drugs can lead to the development of more effective and safer therapeutic agents. It can guide modifications to the chemical structure of drugs to improve their metabolic stability or reduce harmful metabolites.

This also applies to crop protection products such as pesticides, herbicides, fungicides and other crop protection products.

Metabolites may serve as biomarkers of exposure, indicating not just if an organism was exposed to a substance, but also providing information on the amount and timing of exposure. This is particularly useful in epidemiological studies and forensic toxicology.

Regulatory agencies often require a thorough analysis of both the parent compound and its metabolites to evaluate the safety of new drugs, crop protection products, chemicals, and food additives. This is essential for obtaining approval for use and for establishing guidelines for safe exposure levels.

SUMMMARY

The present disclosure provides means for predicting a reaction sequence of a substance.

**In** a first aspect, the present disclosure provides a computer-implemented method, the method comprising:

(a) providing a first model, wherein the first model is configured to determine a first number of reaction products of a reference substance based on a first representation of the reference substance;

(b) providing a second model, wherein the second model is configured to determine a second number of reaction products of the reference substance based on a second representation of the reference substance;

(c) determining or receiving a candidate substance;

(d) inputting a first representation of the candidate substance into the first model;

(e) receiving a first set of predicted reaction products as an output of the first model;

(f) inputting a second representation of the candidate substance into the second model;

(g) receiving a second set of predicted reaction products as an output of the second model;

(h) determining a probability value for each predicted reaction product in the first set and in the second set;

(i) selecting a number of predicted reaction products from the first set and the second set based on their probability values;

(j) repeating steps (d) through (i) for each selected reaction product one or more times to predict one or more further reaction products of one or more further generations;

(k) outputting selected reaction products.

In another aspect, the present disclosure provides a computer system comprising:

a processing unit; and

a memory storing a computer program configured, when executed by the processing unit, to cause the computer system to perform the following:

(a) providing a first model, wherein the first model is configured to determine a first number of reaction products of a reference substance based on a first representation of the reference substance;

(b) providing a second model, wherein the second model is configured to determine a second number of reaction products of the reference substance based on a second representation of the reference substance;

(c) determining or receiving a candidate substance;

(d) inputting a first representation of the candidate substance into the first model;

(e) receiving a first set of predicted reaction products as an output of the first model;

(f) inputting a second representation of the candidate substance into the second model;

(g) receiving a second set of predicted reaction products as an output of the second model;

(h) determining a probability value for each predicted reaction product in the first set and in the second set;

(i) selecting a number of predicted reaction products from the first set and the second set based on their probability values;

(j) repeating steps (d) through (i) for each selected reaction product one or more times to predict one or more further reaction products of one or more further generations;

(k) outputting selected reaction products.

In another aspect, the present disclosure provides a non-transitory computer readable storage medium having stored thereon a computer program that, when executed by a processing unit of a computer system, cause the computer system to execute the following steps:

(a) providing a first model, wherein the first model is configured to determine a first number of reaction products of a reference substance based on a first representation of the reference substance;

(b) providing a second model, wherein the second model is configured to determine a second number of reaction products of the reference substance based on a second representation of the reference substance;

(c) determining or receiving a candidate substance;

(d) inputting a first representation of the candidate substance into the first model;

(e) receiving a first set of predicted reaction products as an output of the first model;

(f) inputting a second representation of the candidate substance into the second model;

(g) receiving a second set of predicted reaction products as an output of the second model;

(h) determining a probability value for each predicted reaction product in the first set and in the second set;

(i) selecting a number of predicted reaction products from the first set and the second set based on their probability values;

(j) repeating steps (d) through (i) for each selected reaction product one or more times to predict one or more further reaction products of one or more further generations;

(k) outputting selected reaction products.

BRIEF DESCRIPTION OF THE DRAWINGS

Fig. 1 shows schematically an example of how a first model is pre-trained.

Fig. 2 shows schematically an example of how a first model is fine-tuned.

Fig. 3 shows schematically a first model is used for prediction.

Fig. 4 shows an example of how a second model is created in the form of a flowchart

Fig. 5 shows schematically an example of how a second model may be structured.

Fig. 6 schematically shows an example of a metabolites prediction for a substance using two models.

Fig. 7 schematically shows an example of a prediction of second-generation reaction products using two models.

Fig. 8 shows an embodiment of the computer-implemented method of the present disclosure in the form of a flow chart.

Fig. 9 illustrates a computer system according to some example implementations of the present disclosure.

DETAILED DESCRIPTION

Various example embodiments will be more particularly elucidated below without distinguishing between the aspects of the disclosure (method, computer system, computer-readable storage medium). On the contrary, the following elucidations are intended to apply analogously to all the aspects of the disclosure, irrespective of in which context (method, computer system, computer-readable storage medium) they occur.

If steps are stated in an order in the present description or in the claims, this does not necessarily mean that the disclosure is restricted to the stated order. On the contrary, it is conceivable that the steps can also be executed in a different order or else in parallel to one another, unless, for example one step builds upon another step, this requiring that the building step be executed subsequently (this being, however, clear in the individual case). The stated orders may thus be exemplary embodiments of the present disclosure.

As used herein, the articles "a" and "an" are intended to include one or more items and may be used interchangeably with "one or more" and "at least one". As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise.

Some implementations of the present disclosure will be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art.

The terms used in this disclosure have the meaning that these terms have in the prior art, in particular in the prior art cited in this disclosure, unless otherwise indicated.

The present disclosure provides means by which a reaction sequence of a candidate substance can be predicted. The reaction sequence is predicted in the form of a set of reaction products of a number of generations.

In other words, the candidate substance may be a starting point of a reaction sequence, and the predicted reaction products may be reaction products in a series of successive chemical reactions in which the reaction product of one reaction becomes the reactant in the next.

If the candidate substance is converted into a first reaction product in a first reaction, this first reaction product is also referred to in this description as a first-generation reaction product. If the first-generation reaction product is converted into a reaction product in a further reaction, this reaction product is also referred to in this disclosure as a second-generation reaction product. The number $N$ of generations thus usually indicates how many reactions lie between the candidate substance and a reaction product under consideration. The number $N$ of generations can be one or two or three or four or five or more than five.

The candidate substance as well as any reaction product is usually a chemical compound. The term "chemical compound" is understood to mean a pure substance consisting of two or more atoms, where (in contrast to mixtures) the atomic species are in a fixed ratio to each other. A chemical compound has a defined chemical structure which reflects the structure at the molecular (or ionic) level.

In an embodiment of the present disclosure, the candidate substance is an organic chemical compound. An "organic chemical compound" is a chemical compound comprising carbon-hydrogen bonds (C-H bonds). In an embodiment of the present disclosure, the candidate substance is an organic chemical compound whose molecules are composed of only the following elements: Carbon (C), Hydrogen (H), Oxygen (O), Nitrogen (N), Sulfur (S), Fluorine (F), Chlorine (Cl), Bromine (Br), Iodine (I) and/or Phosphorus (P).

In an embodiment of the present disclosure, the candidate substance is a xenobiotic for one or more organisms.

In an embodiment of the present disclosure, the candidate substance is an active pharmaceutical ingredient or a metabolite thereof.

In another embodiment of the present disclosure, the candidate substance is an active ingredient of a crop protection product or a metabolite thereof. The crop protection product may be a pesticide, a herbicide, an insecticide or a fungicide or another crop protection product.

In a further embodiment of the present disclosure, one or more reaction products are one or more metabolites of one or more organisms.

In an embodiment of the present disclosure, the organism is a human.

In another embodiment of the present disclosure, the organism is an animal.

In another embodiment of the present disclosure, the organism is a fungus.

In another embodiment of the present disclosure, the organism is a bacterium.

In an embodiment, the organism is a living creature that is commonly used as a laboratory animal in clinical studies. Such an animal may be, for example, a mouse, a rat, a guinea pig, a hamster, a rabbit, a dog, a cat, a pig or a monkey.

In another embodiment, the organism is an organism involved in metabolic studies that are usually carried out for the development and/or market authorization of a new pesticide. Such an organism may be, for example, a fungus, a bacterium, a mouse, a rat, a fish (e.g. zebra fish), a fly (e.g. fruit fly), a frog, a bird, an invertebrate (e.g. earthworm, bee), a cow, a pig, a sheep, or a goat.

In another embodiment, the organism is a collection of organisms or ecological compartments, e.g., in the form of soil, water (e.g., from a water body), sediment, and/or sludge.

The organism may also be a part of an organism, such as tissue, biological fluid, cell culture or one or more enzymes.

In another embodiment of the present disclosure, the candidate substance is a substance that has been synthesized by humans and introduced into the environment.

In another embodiment of the present disclosure, the candidate substance is a substance that occurs in a wastewater, and/or the reaction products are results of treatment of wastewater in a wastewater treatment plant.

In another embodiment of the present disclosure, the candidate substance is a substance that occurs in soil (e.g., contaminated soil), and/or the reaction products are reaction products generated by physical, chemical, and/or biological treatment of the soil.

The prediction of a first-generation reaction product is based on a representation of the candidate substance. The prediction of second-generation reaction product is based on a representation of a first-generation reaction product. The prediction of third-generation reaction product is based on a representation of a second-generation reaction product, and so forth.

The prediction of reaction products is carried out using two or more models. To predict first-generation reaction products, a representation of the candidate substance is inputted into a first model. Furthermore, a representation of the candidate substance is inputted to a second model. If there are more than two models, a representation of the candidate substance is inputted to each of these further models. The representation of a substance inputted into the first model is referred to in this disclosure as the first representation of the substance. The representation of a substance inputted into the second model is referred to in this disclosure as the second representation of the substance. And so forth. However, this does not mean that the representations of the substance inputted into the models are different representations. The representations of the substances inputted to the models may be the same or different. For example, the first representation of a substance and the second representation of the substance may be the same or different. This also applies to representations of reaction products.

Substances and reaction products can be represented in a variety of ways.

Such a representation may include, for example, structural data. Structural data describe how the atoms that make up a substance/reaction product are connected to each other. Structural data describe the molecular structure of the substance/reaction product.

The structural data may be or comprise a molecular graph. A molecular graph is a representation of the structural formula of a substance/reaction product in terms of a mathematical graph. A molecular graph can be a labelled graph whose vertices correspond to the atoms of the compound and edges correspond to chemical bonds. Its vertices can be labelled with the kinds of the corresponding atoms and edges can be labelled with the types of bonds. There are different ways to represent a molecular graph.

Molecular graphs may be represented by matrices, such as an adjacency matrix or by listing for each atom all other atoms it is connected to.

A molecular graph may be represented as a sequence of symbols. An example of such a sequence of symbols is the simplified molecular-input line-entry system (SMILES) representation, which is often stored as an ASCII string.

A molecular graph may be represented as a hierarchy of layers, each layer representing different information of the molecular graph. An example is the IUPAC International Chemical Identifier (InChI), which is often stored as an ASCII string.

A molecular graph may be represented as Wiswesser Line Notation (WLN) representation, which is often stored as an ASCII string.

In an embodiment of the present disclosure, the structural data is or comprises a SMILES representation of the substance/reaction products.

Typically, multiple equally valid SMILES representations can be generated for a substance/reaction product. Therefore, conventions have been defined to ensure that a molecule is always represented by the same SMILES representation (see, e.g., D. Weininger et al.: SMILES. 2nd algorithm for generation of unique SMILES notation, J Chem Inf Comp Sci 1989, 29(2):97e101). Such canonical SMILES representations are unique for each structure.

Another possibility to represent the molecular structure of a substance/reaction product is offered, for example, by the Chemical Markup Language. Chemical Markup Language (CML) is an approach to managing molecular information using tools such as XML (Extended Markup Language) and Java.

The structural data may be or comprise one or more molecular descriptors of the substance/reaction product.

A molecular descriptor is a quantitative representation of a molecule's structure and/or properties that can be used in computational chemistry for understanding and predicting chemical phenomena. These descriptors are crucial in the field of cheminformatics, where they serve as the basis for modelling and analyzing chemical compounds in silico (i.e., through computer simulations).

1D descriptors are a simple form of molecular descriptors, often describing molecular properties that can be represented by a single numerical value, such as molecular weight, number of atoms, or the sum of atomic numbers. 2D descriptors usually take into account the molecular structure or topology, including information about the molecular graph (how atoms are connected to each other). Examples include the Wiener index (a measure of the molecular graph's branching), topological indices, and ring counts. 3D descriptors consider the three-dimensional shape and orientation of molecules, which is crucial for understanding how molecules interact with each other or with biological targets. Examples include steric properties, molecular volume, and surface area. Constitutional descriptors describe the composition and connectivity of atoms within a molecule without considering their 3D arrangement. They include counts of specific atom types, bond types, and/or functional groups. Electronic descriptors are based on the distribution and energy of electrons within a molecule, which are essential for understanding reactivity and chemical behavior. Examples include the highest occupied molecular orbital (HOMO) energy, the lowest unoccupied molecular orbital (LUMO) energy, and dipole moment. Hydrophobicity descriptors quantify the molecule's tendency to interact with or avoid water, which is crucial for understanding solubility and membrane permeability. The logP value, which measures the partition coefficient between octanol and water, is a common hydrophobicity descriptor. Pharmacophore features are features of molecules that are considered crucial for biological activity. They include specific geometric arrangements of atoms or functional groups that are necessary for interaction with a biological target. Molecular descriptors can be generated using the open-source python package RDKit (https://www.rdkit.org), for example.

It is possible that further data, such as physical and/or chemical property data, is used in addition to structural data to reaction products.

Such physical and/or chemical property data can be, for example, molecular weight, water solubility, partition coefficient (e.g., for octanol/water), melting point (e.g., at standard conditions), proportion of polar/apolar functional groups, pKa/pKb values, pH in an aqueous solution and/or other/further physical and/or chemical properties.

The physical and/or chemical property data can be measured values. The physical and/or chemical property data can be read from one or more databases and/or determined from the literature. Examples of databases that store physical and/or chemical properties include PubChem (https://pubchem.ncbi.nlm.nih.gov/), NIST Chemistry WebBook (https://webbook.nist.gov/chemistry/), and CRC Handbook of Chemistry and Physics (https://hbcp.chemnetbase.com/). The physical and/or chemical property data may be determined experimentally.

The physical and/or chemical properties may be calculated values. For example, the physical and/or chemical property data may be calculated based on the molecular structure of the substance/reaction product. There are numerous methods for calculating physical and/or chemical property data based on molecular structure. These can be found in the literature under the name QSPR (Quantitative Structure Property Relationship), among others. There are also commercially and freely available computer programs for calculating physical and/or chemical properties of substances based on their molecular structure (e.g.: QSAR-Co: J. Chem. Inf. Model. 2019, 59, 6, 2538-2544; Molgen-QSPR: https://www.researchgate.net/publication/266470632; RDKit: https://www.rdkit.org).

It is possible that further data, such as data specifying the metabolizing organism, is used in addition to structural data to predict reaction products. It is possible that further data indicate the presence of a certain enzyme and/or co-factor and/or a co-enzyme.

The prediction is made using two models, a first model and a second model. It is possible that the prediction is made using more than two models, e.g., three or four or more than four.

The first model and the second model may be different models. In an embodiment of the present disclosure, the first model and the second model are different models.

The first model and second model can also be sub-models of a single model.

In an embodiment of the present disclosure, the first model and the second model are parts of a model ensemble.

If more than two models are used, they are usually (but not necessarily) different models.

In an embodiment of the present disclosure the first model is a template-free model, and the second model is a template-based model.

Template-based models rely on a predefined library of metabolic reaction templates. These templates are schematic representations of known chemical reactions (e.g., metabolic transformations), detailing how substances are converted into reaction products (e.g., metabolites). A template-based model predicts reaction products by matching the chemical structure of the candidate substance against these templates to find similar known metabolic reactions. When a match is found, the corresponding transformation is applied to the candidate substance to generate a predicted reaction product.

Template-free models, such as sequence-to-sequence or graph-based models, do not rely on predefined templates of chemical reactions. Instead, they utilize machine learning algorithms to learn the patterns of reactant transformation directly from data. These models are trained on training data containing pairs of reference substances and their reactions products, learning to predict the reaction product for a new substance (the candidate substance or an $N^{th}$-generation reaction product, wherein $N$ is an integer greater than zero) based on the learned patterns.

The first model may be a machine learning model.

The term "machine learning" refers to the development of algorithms and statistical models used by computer systems to perform a specific task without explicit instructions, relying instead on patterns and inference. Machine learning is seen as a subset of artificial intelligence. Machine learning algorithms build a mathematical model based on sample data, known as "training data", in order to make predictions or decisions without being explicitly programmed to perform the task.

The term "machine learning model", as used herein, may be understood as a computer implemented data processing architecture. The machine learning model can receive input data and provide output data based on that input data and on parameters of the machine learning model. The machine learning model can learn a relation between input data and output data through training. In training, parameters of the machine learning model are adjusted in order to provide a desired output for a given input.

The process of training a machine learning model involves providing a machine learning algorithm (that is the learning algorithm) with training data to learn from. The term "trained machine learning model" refers to the model artifact that is created by the training process. The training data must contain the correct answer, which is referred to as the target. The learning algorithm finds patterns in the training data that map input data to the target, and it outputs a trained machine learning model that captures these patterns.

In the training process, training data are inputted into the machine learning model and the machine learning model generates an output. The output is compared with the (known) target. Parameters of the machine learning model are modified in order to reduce the deviations between the output and the (known) target to a (defined) minimum.

In general, a loss function can be used for training, where the loss function can quantify the deviations between the output and the target. The aim of the training process can be to modify (adjust) parameters of the machine learning model in order to reduce the loss to a (defined) minimum. The loss function is usually minimized using an optimization method, e.g. a gradient descent method.

Once a machine learning model has been trained, the trained machine learning model can be used for prediction. "Inference" is the process that a trained machine learning model uses to draw conclusions from new data. The term "new" means that this data was not usually used in the training program.

The first model may be configured and trained to predict one or more reaction products for a substance based on a first representation of the substance.

The first model may be a sequence-to-sequence model that takes a first representation of a substance as input data and generates a presentation of one or more reaction products of that substance as output data.

The first model may thus map the representation of a substance onto the representation of one or more reaction products.

The first model may be or comprise a transformer model. A transformer model consists of a specific artificial neural network architecture that was originally developed for use in machine translation and other natural language processing (NLP) tasks.

A transformer model usually comprises an encoder to process the input data and a decoder to generate the output. The task of the encoder is to map an input sequence of embeddings to a sequence of continuous representations, which is then used by the decoder to autoregressively generate an output sequence of tokens. To generate this output sequence the decoder predicts the probability distribution over all tokens in the model's vocabulary at each position in the output sequence based on the input and the preceding tokens in the sequence.

Each encoder and decoder is usually made up of multiple layers that contain attention and feed-forward neural networks. The attention mechanism allows the model to focus on different parts of the input sequence when processing a specific part of that sequence, making it very effective at capturing the context and relationships within the data. The attention mechanism computes attention scores which determine how much each token in the sequence should contribute to the other tokens' representations.

While originally developed for NLP applications, the transformer architecture has since found its way into countless other application areas, such as protein structure prediction and computer-aided synthesis planning (CASP).

The architecture of the first model may be or be based on the Chemformer model introduced by Irwin *et al.* (see, e.g., R. Irwin et al.: Chemformer: a pre-trained transformer for computational chemistry, 2022 Mach. Learn.: Sci. Technol. 3 015022).

The first model may take as input a SMILES representation of a substance and generate SMILES representations for possible reaction products which may be ranked according to their calculated probability.

The input SMILES may be tokenized and then embedded to obtain a numerical representation. To break down the strings into series of tokens a typical strategy is atom-wise tokenisation. With atom-wise tokenization each token corresponds to one atom (hydrogen implicit), bond or other ASCII character denoting the molecule structure of the substance (see, e.g., U.V. Ucak et al.: Improving the quality of chemical language model outcomes with atom-in-SMILES tokenization, Journal of Cheminformatics (2023) 15:55).

The reaction products predicted for a substance are usually specific to a particular scenario and/or use case. Such a scenario and/or use case may be or involve an organism, reaction conditions, available reactants, enzymes and/or the like.

The first model is usually trained on training data that reflects the corresponding scenario and/or use case. However, it is also possible that the first model was trained on training data that covers multiple scenarios and/or use cases. For example, metabolites are usually specific to an organism. Hence, the first model may be trained with training data that

EP 4 787 381 A1

is specific to a particular organism. However, it is also possible that the first model is trained based on training data from a variety of different organisms and then predicts metabolites for different organisms.

In the training of a machine learning model, known methods of data augmentation can be used to increase the diversity of the training data. For example, if the representation of a substance is a SMILES representation, different SMILES that represent the same substance can be generated and used as input data (see, e.g., Igor V. Tetko et al.: State-of-the-art augmented NLP transformer models for direct and single-step retrosynthesis, Nat Commun 11, 5575 (2020)).

The first machine learning model may be trained (or finetuned after pre-training) on training data, wherein the training data comprises, for each reference substance of a plurality of reference substances, (i) a first representation of the reference substance as input data, and (ii) a representation of a reaction product of the reference substance as target data. It is possible that the target data comprise more than one reaction product of the reference substance.

The terms "reference" and "candidate" are used in this disclosure to avoid clarity objections in the patent examination process. The terms "reference" and "candidate" are used in this disclosure to distinguish data used in the training phase from data used in the inference phase. A "reference substance" is a substance from which data is used to train a machine learning model and/or generate a template. A "candidate substance" is a substance from which data is used to predict one or more metabolites using the trained model and/or a template-based model. The terms "reference" and "candidate" have no other limiting meanings. The term "substance" may mean "candidate substance" and/or "reference substance".

Training data can be obtained from public or commercially available databases, such as the Animal Metabolite Database (https://amdb.online/) and/or the LMC Rat Liver Metabolism database (https://oasis-lmc.org/products/data bases/rat-liver-metabolism.aspx).

Training the first machine learning model may include, for each reference substance of the plurality of reference substances:

- inputting the first representation of the reference substance into the first model,

- receiving one or more representations of one or more reaction products of the reference substance as output data from the first model,

- determining a deviation between the output data and the target data,

- reducing the deviation by modifying parameters of the first model.

The deviation between the output data and the target data may be quantified using a cross-entropy loss function, for example.

The training can be carried out until a stop criterion is reached. Such a stop criterion can be for example: a predefined maximum number of training steps/cycles/epochs has been performed, deviations between output data and target data can no longer be reduced by modifying the model parameters, a predefined minimum of the loss function is reached, and/or an extreme value (e.g., maximum or minimum) of another performance value is reached.

The first machine learning model may be pre-trained. The term "pre-trained" refers to a machine learning model that has undergone an initial training phase on a large and diverse dataset before being fine-tuned and/or applied to a specific task. Pre-training involves learning the underlying patterns, structures, and representations of data, which can then be leveraged for various downstream tasks with minimal additional training.

The pre-training may, for example, be done in a self-supervised learning process.

**In** the pre-training, the first model may be trained to reconstruct masked input data.

Taking the tokenized SMILES representation of a substance, short subsequences may be randomly or deterministically replaced with a special token, e.g., denoted as <MASK>. The masked sequences serve as input for the encoder, while the target given to the decoder is the original unmasked token sequence.

Additionally or alternatively, SMILES augmentation may be used as a pre-training task by generating a non-canonical SMILES representation representing the input molecule via random permutation of the atom order and giving it to the model as input. The model may then be asked to predict the canonical form of the SMILES string, which is provided as the target sequence.

The training data for pre-training can come from databases such as ZINC (see, e.g., J. Chem. Inf. Model. 2015, 55, 11, 2324-2337), PubChem (see, e.g., Nucleic Acids Res. 2019 ;47(D1): D1102-D1109) and/or Reaxys (https://www.elsevier.com/products/reaxys).

Additionally or alternatively, the model may be pre-trained to predict one or more molecular descriptors for a reference substance based on structural data of the reference substance (see, e.g., B. Fabian et al.: Molecular representation learning with language models and domain-relevant auxiliary tasks, arXiv:2011.13230v1).

In an embodiment of the present disclosure, the first model was pre-trained to reconstruct masked input data and simultaneously predict molecular descriptors based on structural data. To combine masked input data reconstruction with the prediction of the molecular descriptors an additional regression head may be incorporated into the model structure, which may be composed of one or more fully connected feed-forward layers. The regression head may output predictions for one or more molecular descriptors and may be trained with a Mean-Squared-Error (MSE) or cross-entropy loss function (see, e.g., B. Fabian et al.: Molecular representation learning with language models and domain-relevant auxiliary tasks, arXiv:2011.13230v1).

The first model is usually a template-free model. Unlike template-based methods that rely on known metabolic reactions, template-free approaches learn to predict metabolites directly from data, without predefined templates. Template-free approaches can potentially predict a wider range of metabolic reactions, including novel or less common transformations not covered by existing templates. This flexibility stems from the ability of these methods to learn complex patterns and relationships directly from the data.

Template-free models can automatically learn the rules of biochemical transformations from large datasets of known metabolites and their precursors. This learning process can uncover subtle and complex relationships that might not be immediately apparent to human experts or captured in templates.

Template-free approaches can integrate various types of data, including structural, physicochemical, and biological information, to improve prediction accuracy. This holistic view can provide a more comprehensive understanding of metabolism.

However, template-free models often act as "black boxes", making it challenging to understand how they arrive at their predictions.

The second model may be a template-based model.

Such a template-based model is configured to predict one or more reaction products of a candidate substance based on known reactions of reference substances. This approach is based on the principle that substances that are chemically similar undergo analogous reactions and form analogous reaction products. For example, many metabolic reactions are conserved across species. A candidate substance is likely to be metabolized in a similar way to structurally similar reference substances for which the metabolic fate is known.

The template-based model comprises or has access to a library of known reactions. This library consists of templates, which are essentially representations of reactions (e.g. metabolic transformations). Each template includes the structure of a reference substrate before and after a specific reaction, usually along with reaction conditions, such as the presence of an enzyme or class of enzymes responsible for the reaction. This information may be curated from scientific literature, experimental data, and/or existing databases of metabolic pathways and/or other reactions.

Examples of databases with reactions are Rhea and ChEMBL (see, e.g., P. Bansal et al.: Rhea, the reaction knowledgebase in 2022, Nucleic Acids Research, 2022, Vol. 50, Database issue D693-D700; D. Mendez et al.: ChEMBL: towards direct deposition of bioassay data, Nucleic Acids Res. 2019, 47(D1): D930-D940; E. Coudert et al.: Annotation of biologically relevant ligands in UniProtKB using ChEBI, Bioinformatics, 2023, 39(1), btac793).

The creation of templates, for example, is described in: P. Schwaller et al.: Extraction of organic chemistry grammar from unsupervised learning of chemical reactions, Sci Adv., 2021,7(15): eabe4166; B Delépine et al.: Retro-Path2.0: A retrosynthesis workflow for metabolic engineers, Metabolic Engineering, 2018, 45, 158-170).

Once the library of reaction templates is established, the model matches the candidate substance (the substance whose reaction products is to be predicted) against the templates. This involves comparing the structural data of the candidate substance with those of reference substrates in the templates to find matches. The aim is to identify templates that represent reactions the candidate substance is likely to undergo based on structural similarity.

Such matching algorithms are described in the scientific literature (see, e.g., G. A. Preciat Gonzalez et al.: Comparative evaluation of atom mapping algorithms for balanced metabolic reactions: application to Recon 3D, J Cheminform, 2017, 9(1): 39). Such an algorithm is also implemented in RDKit, for example (see, e.g., RunReactants() in https://www.rdkit.org/RDKit_Docs.2012_12_1.pdf).

In an embodiment of the present disclosure, comparison of the representation of the candidate substance with those of reference substrates is done based on molecular descriptors. Molecular descriptors capture various aspects of molecules. The comparison may thus not (solely) be based on atoms and bonds between atoms, but (also) on physical and/or chemical properties of the molecules (see, e.g., I. Muegge, P. Mukherjee: An overview of molecular fingerprint similarity search in virtual screening, Expert Opinion on Drug Discovery, 2015, 11(2), 137-148).

In an embodiment of the present disclosure, comparison of the representation of the candidate substance with those of reference substrates is done based on molecular fingerprints. Molecular fingerprints are a way to represent molecules as mathematical objects. The most common type of fingerprint is a series of binary digits (bits) that represent the presence or absence of particular substructures in the molecule. Examples of such fingerprints are: Morgan fingerprint, also known as extended-connectivity fingerprint ECFP4, MinHashed fingerprint MHFP6, MAP4, RDKit AP, Molecular ACCess Systems keys fingerprint (MACCS), and PubChem Fingerprints (PubChemFP), AtomPairs2DFingerprint (APFP), GraphOnlyFingerprint (GraphFP), Natural Compound Molecular Fingerprint (NC-MFP) and/or others (see, e.g., A. Capecchi et al.: One molecular fngerprint to rule them all: drugs, biomolecules, and the metabolome, J Cheminform, 2020, 12:43; M. Seo et al.: Development of Natural Compound Molecular Fingerprint (NC-MFP) with the Dictionary of Natural Products (DNP) for natural product-based drug development, J Cheminform, 2020, 12:6).

A similarity coefficient may be calculated to quantify the similarity between the candidate substance and reference substances in the template library. Coefficients for quantifying the similarity can generally be distinguished in similarity metrics and distance or dissimilarity metrics. Examples of similarity metrics are Tanimoto, Dice, and Cosine coefficient; examples of distance metrics are Soergel, Euclidean, and Hamming coefficients. Distance metrics and similarity metrics can easily be interconverted.

For one or more selected templates that match the candidate substance, the transformation represented by the respective template may be applied to the candidate substance to predict the structure of the potential reaction product(s). This step essentially involves modifying the structure of the candidate substance to the metabolic reaction described in the template. An example of software that applies reaction rules to substances is SyGMa (see, e.g., L. Ridder, M. Wagener: SyGMa: combining expert knowledge and empirical scoring in the prediction of metabolites, ChemMedChem 2008, 3(5), 821-832.).

The result is a second set of predicted reaction products, which represent possible outcomes of a chemical reaction of the candidate substance.

The predicted reaction products may then be further refined and validated using various criteria, such as the likelihood of the reaction based on enzyme availability in the organism of interest, the chemical feasibility of the reaction, and/or comparison with experimental data if available. This step may help to prioritize and validate the predictions, making them more reliable.

It is possible that the second model is configured to identify a predefined number of the most similar templates and/or to identify templates for which the similarity is greater than a predefined threshold. It is also possible to select only those metabolites for further processing that are more likely to be formed than a predefined threshold (see, e.g., L. Ridder, M. Wagener: SyGMa: combining expert knowledge and empirical scoring in the prediction of metabolites, ChemMed-Chem 2008, 3(5), 821-832.). Thresholds can be defined by an expert and/or set by a user.

The template-based approach is powerful because it leverages existing knowledge of reactions, making it possible to predict reactions and reaction products of new or understudied substances. For example, it allows to define new templates for all known enzymatic reactions known today: currently there are > 17000 enzymatic reactions originally stored in Rhea listed in the public database UniProt. In addition proprietary enzymatic reactions can be used to define new templates which are used for prediction of metabolites. Predicted reactions products (such as metabolites) can be relatively fast and cost-effective compared to experimental methods. The results of the template-based approach are easier to interpret than the results of purely data-driven models. However, the template-based approach depends on the quality and comprehensiveness of the template library. If a reaction has not been previously characterized or is not included in the library, it won't be predicted. Moreover, the template-based approach may not adequately account for novel or unique reaction pathways that differ significantly from known templates.

As soon as the first model and the second model are set up and configured, they can be used for prediction. The prediction is made for a substance that is referred to in this disclosure as the candidate substance.

The candidate substance is usually selected by a user. However, the candidate substance can also be selected and/or determined automatically.

One or more representations are provided and/or generated for the candidate substance. Such a representation may be or comprise structural data. The structural data may describe the chemical (molecular) structure of the candidate substance.

Such a representation may be received. The term "receiving" is to be interpreted broadly and includes any means capable of providing a representation (or any other data) for further processing by the computer system of the present disclosure. The term "receiving" may mean transmitting data from another computer system to the computer system of the present disclosure. The term "receiving" may mean transmitting data from a device such as a mass spectrometer or a nuclear magnetic resonance (NMR) spectrometer to the computer system of the present disclosure. The term "receiving" may mean uploading of data into the computer system of the present disclosure, e.g. by a user. The term "receiving" may mean retrieving data from a data storage; the data storage may be a component of and/or connected to the computer system of the present disclosure, for example via a network.

Such a representation may be generated (e.g. based on the IUPAC name of the candidate substance).

If further data in addition to a representation of a reference substance was used as input data for training the first model, such further data related to the candidate substance and/or a particular scenario/use case can also be entered into the trained first model.

A first representation of the candidate substance is inputted into the first model.

The trained first model generates a first set of predicted reaction products based on the first representation (and optionally further data) and may output the first set of predicted metabolites. The first set usually contains several reaction products. However, it is also possible that the set only contains one reaction product.

As described above, the first model may be configured to output a set of predicted reaction products together with probability values. Such probability value indicates the probability that a predicted reaction product is an actual reaction product of the candidate substance.

A second representation of the candidate substance is inputted into the second model.

The second model provides, similar to the first model, a set of predicted reaction products, which in this disclosure is referred to as the second set. The second set of predicted reaction products is generated based on the second representation of the candidate substance. The second representation fed to the second model may be the same representation as the first representation that is fed to the first model. However, it is also possible that the second model is supplied with a different representation of the candidate substance than the first model. It is also possible that further data is supplied to the second model as input data. This further data may be the same further data that can optionally be supplied to the first model, or it may be different data.

Both, the template-based approach and the template-free approach may generate some predicted reaction products that are not observed in reality. In other words, both template-based and template-free approaches may predict incorrect reaction products. Usually, predicted reaction products are confirmed experimentally.

For experimental validation, it may be useful to limit the number of predicted reaction products to a smaller number.

The number of predicted reaction products can be reduced based on their probability values. Such a probability value may indicate the probability of a reaction product occurring. Such probability values are usually provided directly by the models that predict reaction products (e.g., by the first model and the second model).

So, in a further step, predicted reaction products are selected from the first set of predicted reaction products (generated by the first model) and from the second set of predicted reaction products (generated by the second model) based on their probability values. If there are more than two models, then each of these further models can generate a further set of predicted reaction products together with corresponding probability values. For the sake of simplicity, the selection is described in more detail on the basis of two sets, without intending to limit the present disclosure to a selection from two sets.

For example, the two sets of predicted reaction products may be merged into a single set and the predicted reaction products in the single set with the highest probability values may be selected. However, it is also possible to consider the two sets individually and select predicted reaction products with the highest probability values from each set.

It is possible to select a predefined number of predicted reaction products with the highest probability, e.g. the top 2 or the top 3 or the top 4 or the top 5 or the top 6 or any other number.

Likewise, it is possible to select those predicted reaction products whose probability values are above a predefined threshold.

The predefined number of reaction products and/or the predefined threshold can, for example, be set by a user.

The selected reaction products are first-generation reaction products. Based on these selected reaction products, second-generation reaction products can be predicted.

This can be done analogously to the process described for predicting first-generation reaction products based on the candidate substance:

In a first step, a first representation is provided for each selected reaction product. The first representation of each selected reaction product is fed to the first model.

Furthermore, a second representation is provided for each selected reaction product and fed to the second model. The first representation and the second representation can be the same or different.

In addition to the representations, further input data can be fed to each model.

The first model generates a first set of predicted reaction products based on the first representation of a first-generation reaction product (and possibly further input data). Typically, the first model also provides a probability value for each predicted reaction product of the second generation.

The second model generates a second set of predicted reaction products based on the second representation of a first-generation reaction product (and possibly further input data). Typically, the second model also provides a probability value for each predicted reaction product of the second generation.

The predicted reaction products of the first set and the second set are second-generation reaction products.

In a further step a probability value for each predicted reaction product in the first set and in the second set is determined.

These probability values may be the probability values provided by the first and second model.

In an embodiment of the present disclosure, each probability value of a second-generation reaction product provided by the first or second model is multiplied by the probability value of that first-generation reaction product of which it is a predicted reaction product.

In other words: if a second-generation product is predicted for a first-generation product, the second-generation product is assigned a probability value that is derived from multiplying the probability value of the first-generation product by the probability value provided by the model for the second-generation reaction product. Generally speaking, if an $n^{th}$-generation product is predicted for an $(n-1)^{th}$-generation product, the $n^{th}$-generation product is assigned a probability value that is derived from the multiplication of the probability value of the $(n\text{-}1)^{th}$-generation product by the probability value provided by the model for the $n^{th}$-generation reaction product, wherein n is an integer greater than 0 and less than or equal to $N$, and $N$ is the number of generations that are predicted. The reaction product of the zeroth generation is the candidate substance, to which a probability value of 1 can be assigned.

In a further step, the reaction products are again selected based on their probability values, as described for the reaction products of the.

In a further step, the reaction products with the highest probability values may be selected as described for the first-generation reaction products.

The number of predicted reaction products to be selected and/or the threshold can be the same as in the case of first-generation reaction products or different. This also applies to all other generations.

The selected second-generation reaction products can in turn be fed to the first model and the second model (and, if available, further models) to predict third-generation reaction products and to make a selection based on the probability values from these predicted third-generation reaction products. The selected third-generation reaction products can in turn be fed to the first model and the second model (and, if available, further models) to predict fourth-generation reaction products and to make a selection based on the probability values from these predicted fourth-generation reaction products. And so on.

The user may specify the number of generations for which predictions are made.

Predicted reaction products can be output. For example, reaction pathways can be output for the candidate substance, comprising predicted reaction products of different generations. Predicted reaction products and/or reaction pathways may be displayed on a monitor, printed out on a printer, stored in a data memory and/or transmitted to a separate computer system.

Predicted reaction products and/or reaction pathways may be experimentally validated and/or validated otherwise, e.g., by experts.

The subject matter of the present disclosure can be used to predict reaction products (e.g., metabolites) for a wide range of different substances and/or organisms and/or scenarios and/or use cases. In this way, the subject matter of the present disclosure can prevent time- and resource-consuming experiments; the number of experiments that need to be carried out to confirm the predictions is significantly reduced.

When developing xenobiotics, metabolites can be predicted at an early stage to identify possible risks arising from metabolites at an early stage.

Metabolite elucidation can be supported by proposing metabolites which might occur in analytics of the chemical composition of organisms treated with candidate products.

The subject matter of the present disclosure will be explained in more detail below with reference to drawings, without the intention of limiting the subject matter of the present disclosures to the features and combinations of features shown in the drawings. Statements made with respect to the embodiments illustrated in the figures are not limited to these embodiments but also apply to and/or in combination with other embodiments.

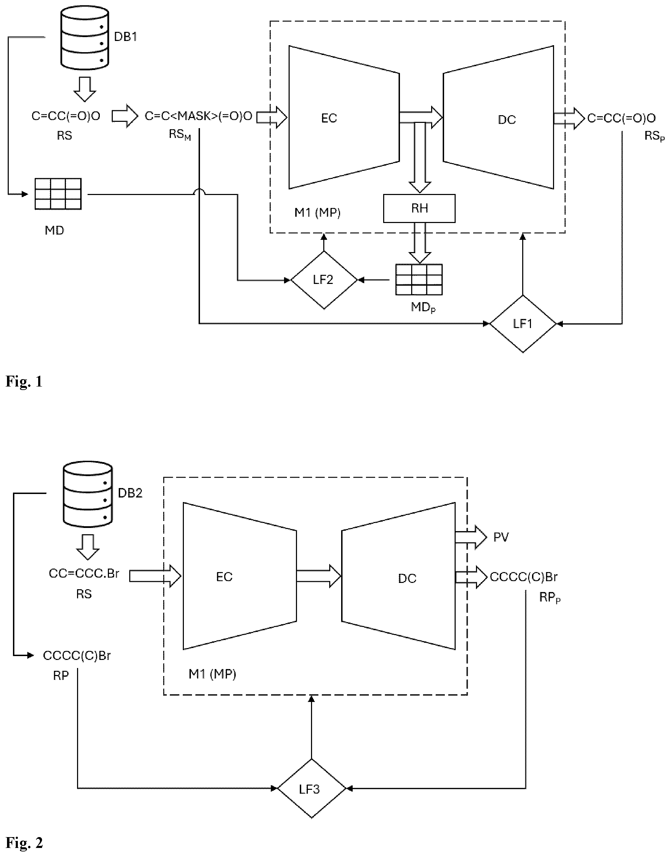

Fig. 1 shows schematically an example of how a first model is pre-trained.

The first model M1 shown in Fig. 1 is a machine learning model. The first model M1 is trained using training data. The training data is obtained from a first database DB1.

The training data comprises, for each reference substance of a plurality of reference substances, (i) a first representation of the reference substance, and (ii) one or more molecular descriptors characterizing the reference substance.

In Fig. 1, only one data set for a single reference substance is shown; usually a plurality of such data sets is used for training. The data set shown comprises a SMILES representation RS of the reference substance and one or more molecular descriptors MD, represented by a table.

Part of the SMILES representation RS of the reference substance is masked. The result is a masked SMILES representation $RS_M$. The masked SMILES representation $RS_M$ is fed into the first model M1. The first model M1 includes an encoder EC, a decoder DC and a regression hat RH.

The primary role of the encoder EC is to process the input sequence (in this case, the masked SMILES representation) and understand the context of each symbol or character within the sequence. It does this through a series of layers, each consisting of multi-head self-attention mechanisms and position-wise fully connected feed-forward networks. The self-attention mechanism allows the model to weight the importance of different parts of the input sequence differently, which is crucial for understanding the context and relationships between different parts of the molecule. The output of the encoder is a set of context-rich embeddings or feature representations that capture the nuances and relationships within the input sequence. The layers described here are not shown in Fig. 1.

The decoder DC, on the other hand, is responsible for generating the output sequence, which in this scenario is the unmasked SMILES representation $RS_P$ of the reference substance. The decoder DC takes the context-rich embeddings from the encoder EC and, starting from an initial token, generates the output sequence one token at a time. The decoder DC also has layers consisting of multi-head self-attention mechanisms, but with a twist: it uses masked attention to prevent future tokens from influencing the prediction of the current token, ensuring that the generation process is autoregressive (i.e., the prediction of each token only depends on previously generated tokens and the input from the encoder). Additionally, the layers of the decoder DC include a second multi-head attention mechanism that focuses on the output of the encoder EC, allowing the decoder DC to utilize the context provided by the encoder EC. Through this process, the decoder DC reconstructs the unmasked SMILES representation from the encoded context. The layers described here are not shown in Fig. 1.

The embeddings generated by the encoder EC are also provided to the regression head RH. The primary job of the regression head RH is to map these embeddings to a continuous output value or values that represent the target prediction, in this scenario one or more predicted molecular descriptors $MD_P$ of the reference substance. The architecture of the regression head may consist of one or more fully connected (dense) layers. The final layer of the regression head RH usually has a number of neurons corresponding to the dimensionality of the output space (one neuron for a single continuous value, or more for multiple continuous values). The layers described here are not shown in Fig. 1.

In the example shown, the unmasked SMILES representation RS of the reference substance and one or more molecular descriptors MD are used as target data. A first loss function LF1 is used to quantify deviations between the unmasked SMILES representation RS and the predicted unmasked SMILES representation $RS_P$. A second loss function LF2 is used to quantify deviations between the one or more molecular descriptors MD and the one or more predicted molecular descriptors $MD_P$.

Deviations can be reduced by modifying model parameters MP of the first model M1. This is usually done in an optimization procedure such as a gradient descent procedure.

The training is carried out for a plurality of data sets from a plurality of reference substances.

The training can be carried out until a stop criterion is reached. Such a stop criterion can be for example: a predefined maximum number of training steps/cycles/epochs has been performed, deviations between output data and target data can no longer be reduced by modifying the model parameters, a predefined minimum of the loss function is reached, and/or an extreme value (e.g., maximum or minimum) of another performance value is reached.

Fig. 2 shows schematically an example of how the first model is fine-tuned.

After the first model M1 shown in Fig. 1 has been pre-trained, it can be trained for the actual task (fine-tuning).

The regression head is no longer required for the actual training; it has been removed; the first model M1 shown in Fig. 2 now only includes the encoder EC and the decoder DC.

The training data is obtained from a second database DB2. In the example shown in Fig. 2, the training data comprises, for each reference substance of a plurality of reference substances, (i) a SMILES representation of the reference substance as input data and one or more SMILES representations of one or more reaction products of the reference substance as target data. In the example shown in Fig. 2, only one data set of a single reference substance is shown. The data set comprises a SMILES representation RS of the reference substance and a SMILES representation RP of a reaction product of the reference substance.

The SMILES representation RS is fed into the first model M1. The first model M1 generates a SMILES representation $RP_P$ of a predicted reaction product based on the SMILES representation RS and model parameters MP. A third loss function LF3 is used to quantify deviations between the SMILES representation RP of the reaction product and the predicted SMILES representation $RP_P$. Deviations can be reduced by modifying model parameters MP of the first model M1.

The training is carried out for a plurality of data sets from a plurality of reference substances.

The training can be carried out until a stop criterion is reached. Such a stop criterion can be for example: a predefined maximum number of training steps/cycles/epochs has been performed, deviations between output data and target data can no longer be reduced by modifying the model parameters, a predefined minimum of the loss function is reached, and/or an extreme value (e.g., maximum or minimum) of another performance value is reached.

Fig. 2 shows that the first model M1 is configured to provide not only the representation $RP_P$ of the predicted reaction product but also a probability value PV. The probability value indicates the probability that the predicted reaction product occurs as a reaction product of the reference substance.

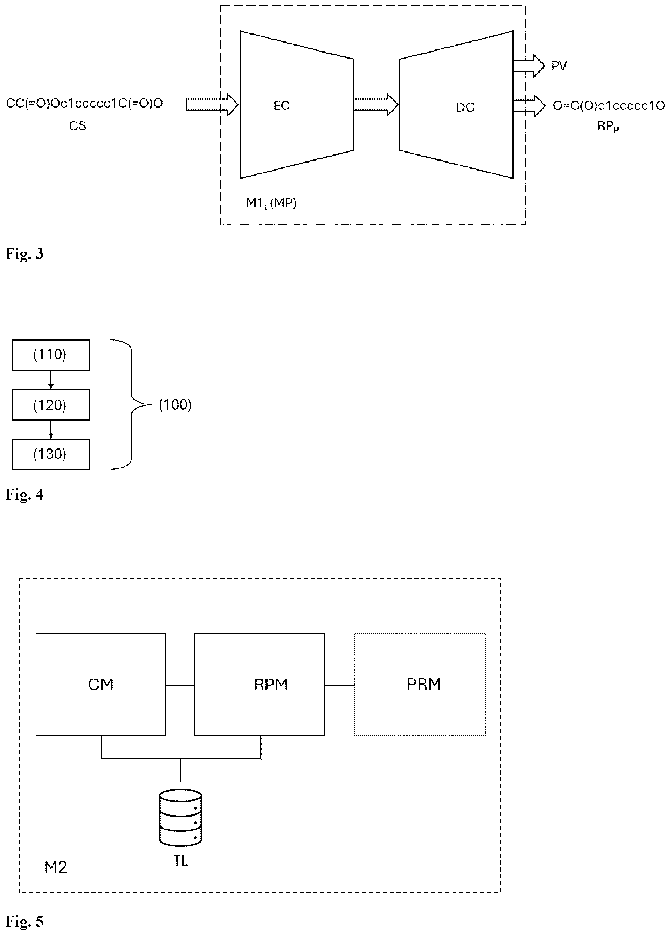

Once the first model has been trained, it can be used for prediction. Fig. 3 schematically shows how the first model is used for prediction.

Fig. 3 shows a trained first model $M1_t$. The trained first model $M1_t$ can be pre-trained and fine-tuned as described in relation to Fig. 1 and Fig. 2.

In the example shown in Fig. 3, the trained first model $M1_t$ is supplied with a SMILES representation CS of a candidate substance. The trained first model $M1_t$ generates a SMILES representation $RP_P$ of a predicted reaction product of the candidate substance based on the SMILES representation CS of the candidate substance.

Fig. 3 shows that the trained first model $M1_t$ is configured to provide not only the representation $RP_P$ of the predicted reaction product but also a probability value PV. The probability value indicates the probability that the predicted reaction product occurs as a reaction product of the candidate substance.

Typically, the trained first model is configured to output a plurality of predicted SMILES representations of a plurality of possible reaction products. The model usually provides a probability value for each predicted reaction product.

As described it is possible to re-input a SMILES representation of a predicted reaction product as input data to the trained first model to predict a next generation of one or more reaction products.

In the examples shown in Fig. 1, Fig. 2 and Fig. 3, the training and prediction is based on SMILES representations. As described in the present disclosure, other structural data than SMILES representations can also be used.

As described, it is also possible to include other/further data in the training.

Fig. 4 shows an example of how a second model can be created in the form of a flowchart. In the example shown in Fig. 4, the second model is a template-based model. In the example shown in Fig. 4, a model is created that can predict metabolites.

The process (100) for creating a second model comprises the following steps:

(110)    selecting an organism or a part thereof,

(120)    identifying and collecting metabolic reactions carried out in or by the selected organism or part thereof,

(130)    creating templates based on the identified and collected metabolic reactions.

The process (100) may include further steps. Each step may include sub-steps.

In step (110), an organism or part of an organism is selected. The organism or part of the organism determines the metabolism on which the second model is based. The part of the organism may be a tissue, a biological fluid, a cell culture or one or more enzymes. It is possible that several organisms and/or parts of organisms will be selected.

In step (120), metabolic reaction carried out in or by the selected organism or part thereof are identified and collected. This information may be curated from scientific literature, experimental data, and/or existing databases of metabolic pathways.

In step (130), templates are created based on the identified and collected metabolic reactions. Such templates are representations of metabolic transformations. Each template includes the structure of a reference substrate before and after a specific metabolic reaction, usually along with the enzyme or class of enzymes responsible for the reaction. The second model is based on these templates.

Fig. 5 shows schematically an example of how a second model may be structured. The second model M2 shown in Fig. 5 is a template-based model.

The second model M2 comprises a template library TL. The template library TL comprises a multitude of (e.g., metabolic) reaction templates. Each template represents a specific type of (e.g. metabolic) reaction, detailing how a particular functional group or molecular structure is transformed (e.g., during metabolism). Templates typically include information about the substrate (the part of the molecule that undergoes the reaction), the product (the outcome of the reaction), and sometimes the enzyme or class of enzymes that catalyze the reaction. This information is usually represented in a generic form that can be applied to a range of structurally similar compounds.

The second model M2 further comprises a compound matching module CM. The compound matching module CM requires structural data of a candidate substance as input data. The compound matching module CM compares the structure of the candidate substance to the substrates in the template library TL to find potential matches. This process usually involves identifying structural features and/or functional groups in the candidate substance that correspond to those in the template substrates.

The second model M2 further comprises a reaction prediction module RPM. For each matching template identified by the compound matching module CM, the reaction prediction module RPM applies the transformation represented by the template to the candidate substance. This involves modifying the chemical structure of the candidate substance according to the reaction detailed in the template to generate predicted reaction products. If the template includes information about enzyme(s) that catalyze the reaction, the reaction prediction module RPM may also consider whether those enzymes are present and active in the organism under consideration to refine the prediction. This step can also be carried out by the prediction refinement module PRM.

The second model M2 may comprise a prediction refinement module PRM. The initial set of predicted reaction products may be refined based on additional information, such as the likelihood of the reaction occurring given the biological context (e.g., liver metabolism in humans) and the chemical feasibility of the predicted transformations.

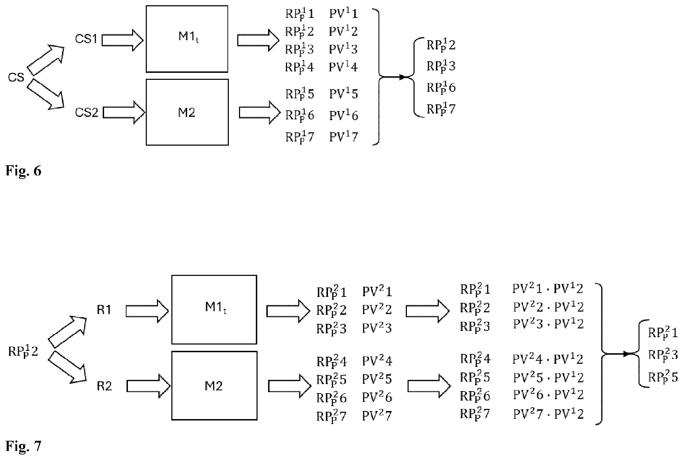

Fig. 6 schematically shows an example of a reaction product prediction for a candidate substance using two models.

The starting point is a candidate substance CS. A first representation CS1 and a second representation CS1 of the candidate substance CS are generated or received. The first representation CS1 and the second representation CS2 may represent the chemical (molecular) structure of the candidate substance CS. The first representation CS1 and the second representation CS2 may be the same or different. The first representation CS1 is fed to a first model $M1_t$; the second representation CS2 is fed to a second model M2.

The first model $M1_t$ and the second model M2 are usually different models. The first model $M1_t$ may be a template-free model; the second model M2 may be a template-based model.

The first model $M1_t$ may, for example, be the model described in relation to Fig. 1, Fig. 2 and Fig. 3. The second model M2 may, for example, be the model described in relation to Fig. 4 and Fig. 5.

The first model $M1_t$ generates a first set of predicted reaction products $RP_P^1 1$, $RP_P^1 2$, $RP_P^1 3$, $RP_P^1 4$ based on the first representation CS1 (and optionally further data). The second model M2 generates a second set of predicted metabolites $RP_P^1 5$, $RP_P^1 6$, $RP_P^1 7$ based on the structural data SD2 (and optionally further data). The superscript [1] indicates that the predicted reaction products are first-generation reaction products. It should be noted that one or more of the reaction products predicted by the first model and by the second model may be the same.

Each of the models provides a probability value for each predicted reaction product. The first model $M1_t$ provides the probability values $PV^1 1$, $PV^1 2$, $PV^1 3$, $PV^1 4$ for the predicted reaction products $RP_P^1 1$, $RP_P^1 2$, $RP_P^1 3$, $RP_P^1 4$. The second model M2 provides the probability values $PV^1 5$, $PV^1 6$, $PV^1 7$ for the predicted reaction products $RP_P^1 5$, $RP_P^1 6$, $RP_P^1 7$.

In a further step, predicted reaction products are selected based on their probability values. For example, it is possible to select a defined number with the highest probability values or to select those reaction predicted products whose probability values are above a threshold. In the example shown in Fig. 6, the reaction products $RP_P^1 1$, $RP_P^1 2$, $RP_P^1 3$, $RP_P^1 4$ are selected.

Each of the selected first-generation reaction products can be fed into the first model $M1_t$ and the second model M2 to predict second-generation reaction products. This is shown in Fig. 7 using the selected reaction product $RP_P^1 2$ as an example.

Fig. 7 schematically shows an example of a prediction of second-generation reaction products using two models.

From the selected first-generation reaction product $RP_P^1 2$, a first representation R1 is inputted into the first model $M1_t$ and a second representation R2 is inputted into the second model M2.

The first model $M1_t$ generates a first set of predicted second-generation reaction products $RP_P^2 1$, $RP_P^2 2$, $RP_P^2 3$ based on the first representation R1 (and optionally further data). The second model M2 generates a second set of predicted metabolites $RP_P^2 4$, $RP_P^2 5$, $RP_P^2 6$, $RP_P^2 7$ based on the second representation R2 (and optionally further data). The superscript [2] indicates that the predicted reaction products are second-generation reaction products. It should be noted that one or more of the reaction products predicted by the first model and by the second model may be the same.

Each of the models provides a probability value for each predicted reaction product. The first model $M1_t$ provides the probability values $PV^2 1$, $PV^2 2$, $PV^2 3$ for the predicted reaction products $RP_P^2 1$, $RP_P^2 2$, $RP_P^2 3$. The second model M2 provides the probability values $PV^2 4$, $PV^2 5$, $PV^2 6$, $PV^2 7$ for the predicted reaction products $RP_P^2 4$, $RP_P^2 5$, $RP_P^2 6$, $RP_P^2 7$.

In the example shown in Fig. 7, the probability values provided by the models are multiplied by the probability value $PV^1 2$ of the first-generation reaction product $RP_P^1 2$ before a selection of second-generation reaction products is made. In the example shown in Fig. 7, the reaction products $RP_P^2 1$, $RP_P^2 3$, $RP_P^3 5$ are selected.

Each of the selected second-generation reaction products can be fed into the first model $M1_t$ and the second model M2 to predict third-generation reaction products. And so on.

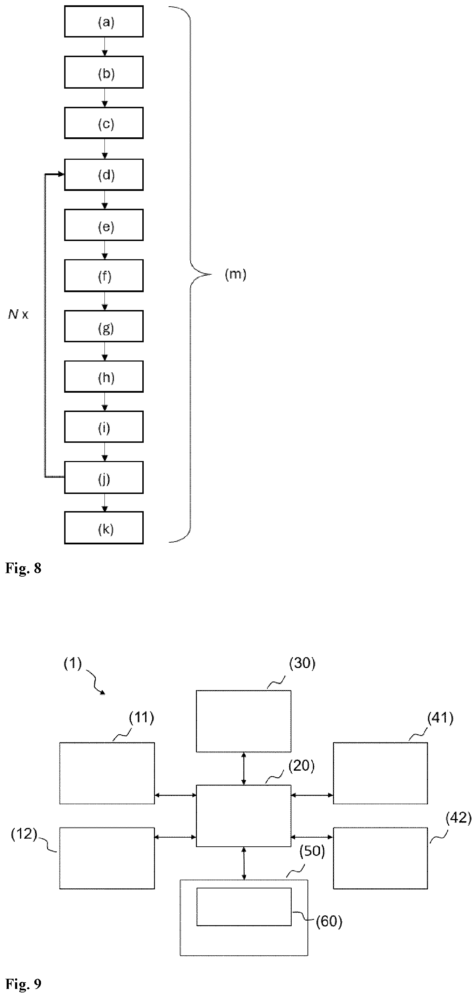

Fig. 8 shows an embodiment of the computer-implemented method of the present disclosure in the form of a flow chart.

The method (m) comprises the following steps:

(a) providing a first model, wherein the first model is configured to determine a first number of reaction products of a reference substance based on a first representation of the reference substance;

(b) providing a second model, wherein the second model is configured to determine a second number of reaction products of the reference substance based on a second representation of the reference substance;

(c) determining or receiving a candidate substance;

(d) inputting a first representation of the candidate substance into the first model;

(e) receiving a first set of predicted reaction products as an output of the first model;

(f) inputting a second representation of the candidate substance into the second model;

(g) receiving a second set of predicted reaction products as an output of the second model;

(h) determining a probability value for each predicted reaction product in the first set and in the second set;

(i) selecting a number of predicted reaction products from the first set and the second set based on their probability values;

(j) repeating steps (d) through (i) for each selected reaction product one or more times to predict one or more further reaction products of one or more further generations;

(k) outputting selected reaction products.

The operations in accordance with the teachings herein may be performed by at least one computer system specially constructed for the desired purposes or general-purpose computer specially configured for the desired purpose by at least one computer program stored in a typically non-transitory computer readable storage medium.

A "computer system" is a system for electronic data processing that processes data by means of programmable calculation rules. Such a system usually comprises a "computer", that unit which comprises a processor for carrying out logical operations, and also peripherals.

In computer technology, "peripherals" refer to all devices which are connected to the computer and serve for the control of the computer and/or as input and output devices. Examples thereof are monitor (screen), printer, scanner, mouse, keyboard, drives, camera, microphone, loudspeaker, etc. Internal ports and expansion cards are, too, considered to be peripherals in computer technology.

Computer systems of today are frequently divided into desktop PCs, portable PCs, laptops, notebooks, netbooks and tablet PCs and so-called handhelds (e.g. smartphone); all these systems can be utilized for carrying out the computer-implemented method of the present disclosure.

The term "non-transitory" is used herein to exclude transitory, propagating signals or waves, but to otherwise include any volatile or non-volatile computer memory technology suitable to the application.

The term "computer system" should be broadly construed to cover any kind of electronic device with data processing capabilities, including, by way of non-limiting example, personal computers, servers, embedded cores, computing system, communication devices, processors (e.g., digital signal processor (DSP)), microcontrollers, field programmable gate array (FPGA), application specific integrated circuit (ASIC), etc.) and other electronic computing devices.

The term "process" as used above is intended to include any type of computation or manipulation or transformation of data represented as physical, e.g., electronic, phenomena which may occur or reside e.g., within registers and/or memories of at least one computer system or processor. The term processing unit includes a single processor or a plurality of distributed or remote such units.

Fig. 9 illustrates a computer system (1) according to some example implementations of the present disclosure in more detail. The computer system may include one or more of each of a number of components such as, for example, a processing unit (20) connected to a memory (50) (e.g., storage device).

The processing unit (20) may be composed of one or more processors alone or in combination with one or more memories. The processing unit (20) is generally any piece of computer hardware that is capable of processing information such as, for example, data, computer programs and/or other suitable electronic information. The processing unit (20) is composed of a collection of electronic circuits some of which may be packaged as an integrated circuit or multiple interconnected integrated circuits (an integrated circuit at times more commonly referred to as a "chip"). The processing unit (20) may be configured to execute computer programs (60), which may be stored onboard the processing unit or otherwise stored in the memory (50) of the same or another computer.

The processing unit (20) may be a number of processors, a multi-core processor or some other type of processor, depending on the particular implementation. Further, the processing unit (20) may be implemented using a number of heterogeneous processor systems in which a main processor is present with one or more secondary processors on a single chip. As another illustrative example, the processing unit (20) may be a symmetric multi-processor system containing multiple processors of the same type. In yet another example, the processing unit (20) may be embodied as or otherwise include one or more ASICs, FPGAs or the like. Thus, although the processing unit (20) may be capable of executing a computer program (60) to perform one or more functions, the processing unit (20) of various examples may be capable of performing one or more functions without the aid of a computer program (60). In either instance, the processing unit (20) may be appropriately programmed to perform functions or operations according to example implementations of the present disclosure.

The memory (50) is generally any piece of computer hardware that is capable of storing information such as, for example, data, images, computer programs (e.g., computer-readable program code (60)), machine learning models and/or other suitable information either on a temporary basis and/or a permanent basis. The memory may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium.

In addition to the memory (50), the processing unit (20) may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s), camera(s) or the like. The communications interface may be configured to transmit and/or receive information by physical (wired) and/or wireless communications links. The communications interface(s) may include interface(s) (41) to connect to a network, such as using technologies such as cellular telephone, Wi-Fi, satellite, cable, digital subscriber line (DSL), fiber optics and the like. In some examples, the communications interface(s) may include one or more short-range communications interfaces (42) configured to connect devices using short-range communications technologies such as NFC, RFID, Bluetooth, Bluetooth LE, ZigBee, infrared (e.g., IrDA) or the like.

The user interfaces may include a display (30). The display (30) may be configured to present or otherwise display information to a user, suitable examples of which include a liquid crystal display (LCD), light-emitting diode display (LED), plasma display panel (PDP) or the like. The user input interface(s) (11) may be wired or wireless, and may be configured to receive information from a user into the computer system (1), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology (12) for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers, cameras and the like.

As indicated above, a computer program (60) may be stored in memory (50), and executed by processing unit (20) that is thereby programmed, to implement functions of the systems, subsystems, tools and their respective elements described herein. As will be appreciated, any suitable program code instructions may be loaded onto a computer or other programmable apparatus from a computer-readable storage medium to produce a particular machine, such that the particular machine becomes a means for implementing the functions specified herein. These program code instructions may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions may produce a computer-implemented process such that the instructions executed by the computer, processing circuitry or other programmable apparatus provide operations for implementing functions described herein.

Execution of instructions by processing unit, or storage of instructions in a computer-readable storage medium, supports combinations of operations for performing the specified functions. In this manner, a computer system (1) may include processing unit (20) and a computer-readable storage medium or memory (50) coupled to the processing circuitry, where the processing circuitry is configured to execute computer-readable program code (60) stored in the memory. It will also be understood that one or more functions, and combinations of functions, may be implemented by special purpose hardware-based computer systems and/or processing circuitry which perform the specified functions, or combinations of special purpose hardware and program code instructions.

**Claims**

1. A computer-implemented method, the method comprising:

   (a) providing a first model, wherein the first model is configured to determine a first number of reaction products of a reference substance based on a first representation of the reference substance;
   (b) providing a second model, wherein the second model is configured to determine a second number of reaction products of the reference substance based on a second representation of the reference substance;
   (c) determining or receiving a candidate substance;
   (d) inputting a first representation of the candidate substance into the first model;
   (e) receiving a first set of predicted reaction products as an output of the first model;
   (f) inputting a second representation of the candidate substance into the second model;
   (g) receiving a second set of predicted reaction products as an output of the second model;
   (h) determining a probability value for each predicted reaction product in the first set and in the second set;
   (i) selecting a number of predicted reaction products from the first set and the second set based on their probability values;
   (j) repeating steps (d) through (i) for each selected reaction product one or more times to predict one or more further reaction products of one or more further generations;
   (k) outputting selected reaction products.

2. The method of claim 1, wherein the first model is a template-free machine learning model, and the second model is a template-based model.

3. The method of claim 1 or 2, wherein the candidate substance is a xenobiotic.

4. The method of any one of claims 1 to 3, wherein the candidate substance is a crop protection product.

5. The method of any one of claims 1 to 3, wherein the candidate substance is a pharmaceutical.

6. The method of any one of claims 1 to 5, wherein each predicted reaction product is a potential metabolite of one or more organisms or part thereof.

7. The method of any one of claims 1 to 6, wherein selecting a number of predicted reaction products from the first set and the second set based on their probability values comprises:

   - selecting a predefined number of predicted reaction products with the highest probability values.

8. The method of any one of claims 1 to 7, wherein selecting a number of predicted reaction products from the first set and the second set based on their probability values comprises:

   - selecting those predicted reaction products whose probability values are greater than a predefined threshold.

9. The method of any one of claims 1 to 8, wherein repeating steps (d) through (i) means:

   - performing steps (d) through (i) for each selected reaction product of generation n, wherein a first representation of the reaction product of generation n is supplied to the first model and the first model provides a first set of predicted reaction products of generation $n+1$, wherein a second representation of the reaction product of generation n is supplied to the second model and the second model provides a second set of reaction products of generation $n+1$, where n is an integer greater than 0.

10. The method of any one of claims 1 to 9, wherein the first model and the second model are configured to provide a probability value for each predicted reaction product, such probability value indicating the probability of the predicted reaction product occurring.

11. The method of any one of claims 1 to 10, wherein determining a probability value for each predicted reaction product in the first set and in the second set comprises:

- multiplying the probability value of a reaction product of generation n provided by the first model or the second model by the probability value of the reaction product of generation n-1, on which the predicted reaction product of generation n is based, wherein the reaction product of generation n-1=0 is the candidate substance to which a probability value of 1 is assigned, wherein n is an integer greater than 0.

12. The method of any one of claims 1 to 11, wherein the first representation of the candidate substance and/or the second representation of the candidate substance comprise(s) structural data of the candidate substance.

13. The method of any one of claims 1 to 12, wherein the candidate is a xenobiotic for one or more organisms, wherein the one or more organisms comprise is an animal or a fungus.

14. A computer system comprising:

a processing unit; and
a memory storing a computer program configured, when executed by the processing unit, to cause the computer system to perform the following:

(a) providing a first model, wherein the first model is configured to determine a first number of reaction products of a reference substance based on a first representation of the reference substance;
(b) providing a second model, wherein the second model is configured to determine a second number of reaction products of the reference substance based on a second representation of the reference substance;
(c) determining or receiving a candidate substance;
(d) inputting a first representation of the candidate substance into the first model;
(e) receiving a first set of predicted reaction products as an output of the first model;
(f) inputting a second representation of the candidate substance into the second model;
(g) receiving a second set of predicted reaction products as an output of the second model;
(h) determining a probability value for each predicted reaction product in the first set and in the second set;
(i) selecting a number of predicted reaction products from the first set and the second set based on their probability values;
(j) repeating steps (d) through (i) for each selected reaction product one or more times to predict one or more further reaction products of one or more further generations;
(k) outputting selected reaction products.

15. A non-transitory computer readable storage medium having stored thereon a computer program that, when executed by a processing unit of a computer system, cause the computer system to execute the following steps:

(a) providing a first model, wherein the first model is configured to determine a first number of reaction products of a reference substance based on a first representation of the reference substance;
(b) providing a second model, wherein the second model is configured to determine a second number of reaction products of the reference substance based on a second representation of the reference substance;
(c) determining or receiving a candidate substance;
(d) inputting a first representation of the candidate substance into the first model;
(e) receiving a first set of predicted reaction products as an output of the first model;
(f) inputting a second representation of the candidate substance into the second model;
(g) receiving a second set of predicted reaction products as an output of the second model;
(h) determining a probability value for each predicted reaction product in the first set and in the second set;
(i) selecting a number of predicted reaction products from the first set and the second set based on their probability values;
(j) repeating steps (d) through (i) for each selected reaction product one or more times to predict one or more further reaction products of one or more further generations;
(k) outputting selected reaction products.

**Amended claims in accordance with Rule 137(2) EPC.**

1. A computer-implemented method, the method comprising:

(a) providing a first model ($M1_t$), wherein the first model ($M1_t$) is configured to determine a first number of reaction products ($RP_p$) of a reference substance based on a first representation (RS) of the reference substance;

(b) providing a second model (M2), wherein the second model (M2) is configured to determine a second number of reaction products of the reference substance based on a second representation of the reference substance;

(c) determining or receiving a candidate substance;

(d) inputting a first representation (CS1) of the candidate substance into the first model ($M1_t$);

(e) receiving a first set of predicted reaction products ( $RP_P^1 1$, $RP_P^1 2$, $RP_P^1 3$, $RP_P^1 4$ ) as an output of the first model ($M1_t$);

(f) inputting a second representation (CS2) of the candidate substance into the second model (M2);

(g) receiving a second set of predicted reaction products ( $RP_P^1 5$, $RP_P^1 6$, $RP_P^1 7$ ) as an output of the second model (M2);

(h) determining a probability value ($PV^1 1$, $PV^1 2$, $PV^1 3$, $PV^1 4$, $PV^1 5$, $PV^1 6$, $PV^1 7$) for each predicted reaction product ( $RP_P^1 1$, $RP_P^1 2$, $RP_P^1 3$, $RP_P^1 4$, $RP_P^1 5$, $RP_P^1 6$, $RP_P^1 7$ ) in the first set and in the second set;

(i) selecting a number of predicted reaction products ( $RP_P^1 2$, $RP_P^1 3$, $RP_P^1 6$, $RP_P^1 7$ ) from the first set and the second set based on their probability values ($PV^1 2$, $PV^1 3$, $PV^1 6$, $PV^1 7$);

(j) repeating steps (d) through (i) for each selected reaction product ( $RP_P^1 2$, $RP_P^1 3$, $RP_P^1 6$, $RP_P^1 7$ ) one or more times to predict one or more further reaction products ( $RP_P^2 1$, $RP_P^2 2$, $RP_P^2 3$, $RP_P^2 4$, $RP_P^2 5$, $RP_P^2 6$, $RP_P^2 7$ ) of one or more further generations;

(k) outputting selected reaction products.

2. The method of claim 1, wherein the first model ($M1_t$) is a template-free machine learning model, and the second model (M2) is a template-based model.

3. The method of claim 1 or 2, wherein the candidate substance is a xenobiotic.

4. The method of any one of claims 1 to 3, wherein the candidate substance is a crop protection product.

5. The method of any one of claims 1 to 3, wherein the candidate substance is a pharmaceutical.

6. The method of any one of claims 1 to 5, wherein each predicted reaction product ( $RP_P^1 1$, $RP_P^1 2$, $RP_P^1 3$, $RP_P^1 4$, $RP_P^1 5$, $RP_P^1 6$, $RP_P^1 7$, $RP_P^2 1$, $RP_P^2 2$, $RP_P^2 3$, $RP_P^2 4$, $RP_P^2 5$, $RP_P^2 6$, $RP_P^2 7$ ) is a potential metabolite of one or more organisms or part thereof.

7. The method of any one of claims 1 to 6, wherein selecting a number of predicted reaction products ( $RP_P^1 2$, $RP_P^1 3$, $RP_P^1 6$, $RP_P^1 7$ ) from the first set and the second set based on their probability values ($PV^1 2$, $PV^1 3$, $PV^1 6$, $PV^1 7$) comprises:

- selecting a predefined number of predicted reaction products with the highest probability values.

8. The method of any one of claims 1 to 7, wherein selecting a number of predicted reaction products ( $RP_P^1 2$, $RP_P^1 3$, $RP_P^1 6$, $RP_P^1 7$ ) from the first set and the second set based on their probability values ($PV^1 2$, $PV^1 3$, $PV^1 6$, $PV^1 7$); comprises:

- selecting those predicted reaction products whose probability values are greater than a predefined threshold.

9. The method of any one of claims 1 to 8, wherein repeating steps (d) through (i) means:

- performing steps (d) through (i) for each selected reaction product ( $RP_P^1 2$ ) of generation *n,* wherein a first representation (R1) of the reaction product ( $RP_P^1 2$ ) of generation *n* is supplied to the first model ($M1_t$) and the first model ($M1_t$) provides a first set of predicted reaction products ( $RP_P^2 1$, $RP_P^2 2$, $RP_P^2 3$ ) of generation *n*+1, wherein a second representation (R2) of the reaction product ( $RP_P^1 2$ ) of generation n is supplied to the second model (M2) and the second model (M2) provides a second set of reaction products ( $RP_P^2 4, RP_P^2 5, RP_P^2 6, RP_P^2 7$ ) of generation $n$+1, where $n$ is an integer greater than 0.

10. The method of any one of claims 1 to 9, wherein the first model (M1$_t$) and the second model (M2) are configured to provide a probability value (PV$^1$1, PV$^1$2, PV$^1$3, PV$^1$4, PV$^1$5, PV$^1$6, PV$^1$7, PV$^2$1, PV$^2$2, PV$^2$3, PV$^2$4, PV$^2$5, PV$^2$6, PV$^2$7) for each predicted reaction product ( $RP_P^1 1, RP_P^1 2, RP_P^1 3, RP_P^1 4$ , $RP_P^1 5, RP_P^1 6, RP_P^1 7, RP_P^2 1, RP_P^2 2, RP_P^2 3, RP_P^2 4, RP_P^2 5, RP_P^2 6, RP_P^2 7$ ), such probability value (PV$^1$1, PV$^1$2, PV$^1$3, PV$^1$4, PV$^1$5, PV$^1$6, PV$^1$7, PV$^2$1, PV$^2$2, PV$^2$3, PV$^2$4, PV$^2$5, PV$^2$6, PV$^2$7) indicating the probability of the predicted reaction product ( $RP_P^1 1, RP_P^1 2, RP_P^1 3, RP_P^1 4, RP_P^1 5, RP_P^1 6, RP_P^1 7, RP_P^2 1$ , $RP_P^2 2, RP_P^2 3, RP_P^2 4, RP_P^2 5, RP_P^2 6, RP_P^2 7$ ) occurring.

11. The method of any one of claims 1 to 10, wherein determining a probability value (PV$^1$1, PV$^1$2, PV$^1$3, PV$^1$4, PV$^1$5, PV$^1$6, PV$^1$7, PV$^2$1, PV$^2$2, PV$^2$3, PV$^2$4, PV$^2$5, PV$^2$6, PV$^2$7) for each predicted reaction product ( $RP_P^1 1, RP_P^1 2, RP_P^1 3, RP_P^1 4, RP_P^1 5, RP_P^1 6, RP_P^1 7, RP_P^2 1, RP_P^2 2, RP_P^2 3, RP_P^2 4, RP_P^2 5$ , $RP_P^2 6, RP_P^2 7$ ) in the first set and in the second set comprises:

- multiplying the probability value (PV$^2$1, PV$^2$2, PV$^2$3, PV$^2$4, PV$^2$5, PV$^2$6, PV$^2$7) of a reaction product ( $RP_P^2 1, RP_P^2 2, RP_P^2 3, RP_P^2 4, RP_P^2 5, RP_P^2 6, RP_P^2 7$ ) of generation $n$ provided by the first model (M1$_t$) or the second model (M2) by the probability value (PV$^1$2) of the reaction product ( $RP_P^1 2$ ) of generation $n$-1, on which the predicted reaction product ( $RP_P^2 1, RP_P^2 2, RP_P^2 3, RP_P^2 4, RP_P^2 5, RP_P^2 6, RP_P^2 7$ ) of generation n is based, wherein the reaction product of generation $n$-1=0 is the candidate substance to which a probability value of 1 is assigned, wherein $n$ is an integer greater than 0.

12. The method of any one of claims 1 to 11, wherein the first representation (CS1) of the candidate substance and/or the second representation (CS2) of the candidate substance comprise(s) structural data of the candidate substance.

13. The method of any one of claims 1 to 12, wherein the candidate is a xenobiotic for one or more organisms, wherein the one or more organisms comprise is an animal or a fungus.

14. A computer system (1) comprising:

a processing unit (20); and
a memory (50) storing a computer program (60) configured, when executed by the processing unit (20), to cause the computer system (1) to perform the following:

(a) providing a first model (M1$_t$), wherein the first model (M1$_t$) is configured to determine a first number of reaction products (RP$_p$) of a reference substance based on a first representation (RS) of the reference substance;
(b) providing a second model (M2), wherein the second model (M2) is configured to determine a second number of reaction products of the reference substance based on a second representation of the reference substance;
(c) determining or receiving a candidate substance;
(d) inputting a first representation (CS1) of the candidate substance into the first model (M1$_t$);
(e) receiving a first set of predicted reaction products ( $RP_P^1 1, RP_P^1 2, RP_P^1 3, RP_P^1 4$ ) as an output of the first model (M1$_t$);
(f) inputting a second representation (CS2) of the candidate substance into the second model (M2);
(g) receiving a second set of predicted reaction products ( $RP_P^1 5, RP_P^1 6, RP_P^1 7$ ) as an output of the second model (M2);
(h) determining a probability value (PV$^1$1, PV$^1$2, PV$^1$3, PV$^1$4, PV$^1$5, PV$^1$6, PV$^1$7) for each predicted reaction product ( $RP_P^1 1, RP_P^1 2, RP_P^1 3, RP_P^1 4, RP_P^1 5, RP_P^1 6, RP_P^1 7$ ) in the first set and in the second set;

(i) selecting a number of predicted reaction products ( $RP_P^1 2$, $RP_P^1 3$, $RP_P^1 6$, $RP_P^1 7$ ) from the first set and the second set based on their probability values ($PV^1 2$, $PV^1 3$, $PV^1 6$, $PV^1 7$);

(j) repeating steps (d) through (i) for each selected reaction product ( $RP_P^1 2$, $RP_P^1 3$, $RP_P^1 6$, $RP_P^1 7$ ) one or more times to predict one or more further reaction products ( $RP_P^2 1$, $RP_P^2 2$, $RP_P^2 3$, $RP_P^2 4$, $RP_P^2 5$, $RP_P^2 6$, $RP_P^2 7$ ) of one or more further generations;

(k) outputting selected reaction products.

15. A non-transitory computer readable storage medium having stored thereon a computer program (60) that, when executed by a processing unit (20) of a computer system (1), cause the computer system (1) to execute the following steps:

(a) providing a first model ($M1_t$), wherein the first model ($M1_t$) is configured to determine a first number of reaction products ($RP_p$) of a reference substance based on a first representation (RS) of the reference substance;
(b) providing a second model (M2), wherein the second model (M2) is configured to determine a second number of reaction products of the reference substance based on a second representation of the reference substance;
(c) determining or receiving a candidate substance;
(d) inputting a first representation (CS1) of the candidate substance into the first model ($M1_t$);

(e) receiving a first set of predicted reaction products ( is an animal or a fungus. ) as an output of the first model ($M1_t$);
(f) inputting a second representation (CS2) of the candidate substance into the second model (M2);

(g) receiving a second set of predicted reaction products ( ) as an output of the second model (M2);
(h) determining a probability value ($PV^1 1$, $PV^1 2$, $PV^1 3$, $PV^1 4$, $PV^1 5$, $PV^1 6$, $PV^1 7$) for each predicted reaction product ( puter system (1) to perform the following: ) in the first set and in the second set;

(i) selecting a number of predicted reaction products ( ice based on a first repre ) from the first set and the second set based on their probability values ($PV^1 2$, $PV^1 3$, $PV^1 6$, $PV^1 7$);

(j) repeating steps (d) through (i) for each selected reaction product ( configured to determine a ) one or more times to predict one or more further reaction products ( ased on a second representation of the refere ) of one or more further generations;
(k) outputting selected reaction products.

Fig. 1

Fig. 2

CC(=O)Oc1ccccc1C(=O)O
CS

PV

O=C(O)c1ccccc1O
$RP_p$

EC

DC

$M1_t$ (MP)

**Fig. 3**

(110)

(120)

(130)

(100)

**Fig. 4**

CM

RPM

PRM

TL

M2

**Fig. 5**

Fig. 6

Fig. 7

Fig. 8

Fig. 9

Europäisches
Patentamt

European
Patent Office

Office européen
des brevets

## EUROPEAN SEARCH REPORT

**Application Number**

EP 25 15 4581

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Jin Wengong ET AL: "Predicting Organic Reaction Outcomes with Weisfeiler-Lehman Network", <br><br> , <br> 29 December 2017 (2017-12-29), pages 1-11, XP093294690, <br> Retrieved from the Internet: <br> URL:https://arxiv.org/pdf/1709.04555 | 1-5, 7-12,14, 15 | INV. <br> G16C20/10 |
| Y | * 2 Related Work <br> 5 Conclusion * <br> * abstract * <br> * Setup for Candidate Ranking; page 7 * <br> ----- | 6,13 | |
| Y | HUGHES TYLER B ET AL: "Metabolic Forest: Predicting the Diverse Structures of Drug Metabolites", <br> JOURNAL OF CHEMICAL INFORMATION AND MODELING, <br> vol. 60, no. 10, <br> 1 October 2020 (2020-10-01), pages 4702-4716, XP093294664, <br> US <br> ISSN: 1549-9596, DOI: 10.1021/acs.jcim.0c00360 <br> Retrieved from the Internet: <br> URL:https://pubs.acs.org/doi/pdf/10.1021/acs.jcim.0c00360?ref=article_openPDF> <br> * abstract * <br> * CONCLUSIONS * <br> ----- | 6,13 | TECHNICAL FIELDS SEARCHED (IPC) <br><br> G16C |

The present search report has been drawn up for all claims

| Place of search | Date of completion of the search | Examiner |
|---|---|---|
| Munich | 11 July 2025 | Beligny, Samuel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 .......................................................................

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **P.G. RIEGER et al.** Xenobiotics in the environment: present and future strategies to obviate the problem of biological persistence. *Journal of Biotechnology*, 2002, vol. 94 (1), 101-123
- **D. WEININGER et al.** SMILES. 2nd algorithm for generation of unique SMILES notation. *J Chem Inf Comp Sci*, 1989, vol. 29 (2), 97-101
- **QSAR-CO**. *J. Chem. Inf. Model.*, 2019, vol. 59 (6), 2538-2544
- **R. IRWIN et al.** Chemformer: a pre-trained transformer for computational chemistry. *Mach. Learn.: Sci. Technol.*, 2022, vol. 3, 015022
- **U.V. UCAK et al.** Improving the quality of chemical language model outcomes with atom-in-SMILES tokenization. *Journal of Cheminformatics*, 2023, vol. 15, 55
- **IGOR V. TETKO et al.** State-of-the-art augmented NLP transformer models for direct and single-step retrosynthesis. *Nat Commun*, 2020, vol. 11, 5575
- *J. Chem. Inf. Model.*, 2015, vol. 55 (11), 2324-2337
- *Nucleic Acids Res.*, 2019, vol. 47 (D1), 1102-1109
- **B. FABIAN et al.** Molecular representation learning with language models and domain-relevant auxiliary tasks. *arXiv:2011.13230v1*
- **P. BANSAL et al.** Rhea, the reaction knowledgebase. *Nucleic Acids Research*, 2022, vol. 50
- **D. MENDEZ et al.** ChEMBL: towards direct deposition of bioassay data. *Nucleic Acids Res.*, 2019, vol. 47 (D1), 930-940

- **E. COUDERT et al.** Annotation of biologically relevant ligands in UniProtKB using ChEBI. *Bioinformatics*, 2023, vol. 39 (1), 793
- **P. SCHWALLER et al.** Extraction of organic chemistry grammar from unsupervised learning of chemical reactions. *Sci Adv.*, 2021, vol. 7 (15), 4166
- **B DELÉPINE et al.** RetroPath2.0: A retrosynthesis workflow for metabolic engineers. *Metabolic Engineering*, 2018, vol. 45, 158-170
- **G. A. PRECIAT GONZALEZ et al.** Comparative evaluation of atom mapping algorithms for balanced metabolic reactions: application to Recon 3D. *J Cheminform*, 2017, vol. 9 (1), 39
- **I. MUEGGE ; P. MUKHERJEE**. An overview of molecular fingerprint similarity search in virtual screening. *Expert Opinion on Drug Discovery*, 2015, vol. 11 (2), 137-148
- **A. CAPECCHI et al.** One molecular fngerprint to rule them all: drugs, biomolecules, and the metabolome. *J Cheminform*, 2020, vol. 12, 43
- **M. SEO et al.** Development of Natural Compound Molecular Fingerprint (NC-MFP) with the Dictionary of Natural Products (DNP) for natural product-based drug development. *J Cheminform*, 2020, vol. 12, 6
- **L. RIDDER ; M. WAGENER**. SyGMa: combining expert knowledge and empirical scoring in the prediction of metabolites. *ChemMedChem*, 2008, vol. 3 (5), 821-832